# EUROPÄISCHE PATENTSCHRIFT

(11) **EP 1 153 293 B1**
(45) Veröffentlichungstag und Bekanntmachung des Hinweises auf die Patenterteilung: **14.08.2002**
(21) Anmeldenummer: 00915148.1
(22) Anmeldetag: 14.02.2000
(51) Int. Cl.: G01N 33/24, G01N 33/18, G01N 15/08

(54) **LYSIMETERSTATION**
LYSIMETER STATION
STATION LYSIM TRIQUE

(30) Priorität: 13.02.1999 DE 19907463
(43) Veröffentlichungstag der Anmeldung: 14.11.2001
(73) Patentinhaber: UFZ-UMWELTFORSCHUNGSZENTRUM Leipzig-Halle GmbH, 04318 Leipzig (DE); Umwelt-Geräte-Technik GmbH, 15474 Müncheberg (DE); Kunststoff-Systeme Bräutigam GmbH, 07973 Greiz (DE)
(72) Erfinder: MEISSNER, Ralph, D-39615 Seehausen (DE); SEYFARTH, Manfred, D-12559 Berlin (DE); BRÄUTIGAM, Roland, D-07973 Greiz (DE); RUPP, Holger, D-39615 Seehausen (DE)
(74) Vertreter: Hengelhaupt, Jürgen D., Dipl.-Ing.
(86) Internationale Anmeldenummer: EP0001161
(87) Internationale Veröffentlichungsnummer: WO0047994

(56) Entgegenhaltungen:
- WO-A-95/22750
- DE-A- 3 432 264
- DE-A- 3 706 479
- DE-C- 19 510 917
- US-A- 4 759 227
- US-A- 5 594 185

## Beschreibung

Die Erfindung betrifft eine Lysimeterstation gemäß dem Oberbegriff des Anspruchs 1.

Lysimeter sind Meßeinrichtungen zur Bestimmung des Wasser- oder Stoffhaushaltes eines Bodenkörpers unter vorgegebenen Bedingungen.

In der Regel sollen dabei die natürlichen Bedingungen eines gewählten Standortes realisiert werden. Zur Realisierung dieser Bedingungen werden die Lysimetergefäße direkt auf dem zu untersuchenden Standort installiert. Damit können die geforderten Randbedingungen an der Oberfläche des betrachteten Bodenkörpers weitgehend erfüllt werden. Die untere Randbedingung des Bodenkörpers im Lysimeter muß in geeigneter Weise geführt werden, so daß die Wasserbewegung in gleicher Weise erfolgt wie im ungestörten Boden (Deutscher Verband für Wasserwirtschaft und Kulturbau e.V.(1980): Empfehlungen zum Bau und Betrieb von Lysimetern,Heft 114).

Für den Betrieb von Lysimetern ist eine Zugangsmöglichkeit zu dem mit Boden gefüllten Lysimetergefäß erforderlich. Dieser Zugang dient der Funktionskontrolle sowie der Aufnahme der Meß-, Steuerungs- und Wägetechnik. Der Zugang wird bisher in Form von Lysimeterkellern oder Lysimeterschächten errichtet, wofür meist aufwendige Beton-, Stahl- oder Stahlbetonkonstruktionen erforderlich sind. Diese Konstruktionen müssen gegen den Eintritt von Niederschlags-, Stau- und Grundwasser abgedichtet werden, um einen störungsfreien Betrieb der Lysimetermessungen sowie der Funktionstüchtigkeit der peripheren Meßsensorik zu gewährleisten. Konventionelle Lysimeterkeller oder -schächte müssen zu diesem Zweck mit hohem technischen und materiellen Aufwand abgedichtet werden.

Der Bau auf grundwassernahen Standorten oder Standorten mit hoher Aggressivität gegenüber Stahl und Beton (zum Beispiel Deponien, Bergbaufolgeflächen) ist dabei besonders kostenintensiv, da oftmals eine langfristige Wasserhaltung während der Bauausführung und teilweise auch während des Betriebs erforderlich ist. Aufgrund der hohen Kosten für die Wasserhaltung und den Betrieb werden in situ Lysimeteranlagen auf grundwasserbeeinflußten Standorten bzw. Standorten mit hoher Aggressivität gegenüber Stahl und Beton nur in Ausnahmefällen errichtet.

Der Erfindung liegt die Aufgabe zugrunde, eine gattungsgemäße Lysimeterstation zu entwickeln, die kostengünstiger ausgeführt werden kann, die von der Standortbeschaffenheit unabhängig einsetzbar und gegen den Eintritt von Niederschlags-, Stau- und Grundwasser zuverlässig ohne zusätzliche Maßnahmen abgedichtet ist und mit der ein zuverlässiger Betrieb der Lysimetermessungen und der peripheren Meßtechnik gewährleistet wird.

Erfindungsgemäß wird diese Aufgabe durch die Merkmale des Anspruches 1 gelöst. Nach der Erfindung wird der Zugang für die Funktionskontrolle sowie für die Aufnahme der Meß-, Steuerungs- und Wägetechnik mit mindestens einem Lysimetergefäß einstückig als oben offener Behälter ausgeführt.

Gegenüber konventionellen Lysimeterkellern oder -schächten wird ein erheblicher Kostenvorteil erzielt, da die komplette Anordnung als geschlossene Einheit in Form eines Containers mit auf den Anwendungsfall zugeschnittener variabler Größe an einem beliebigen Standort installiert werden kann.

Durch die Verwendung von inertem Kunststoff mit hermetischer Boden- und Seitenabdichtung ist der Einsatz derartiger Lysimeteranordnungen auch auf Extremstandorten mit hohem Grundwasserstand, auf Deponien und auf Standorten mit stahl- und betonaggressiven Wässern einsetzbar.

Vorteilhafte Weiterbildungen der Erfindung ergeben sich aus den Unteransprüchen.

Die Erfindung wird nachstehend an einem Ausführungsbeispiel eine Lysimeterstation mit vier Lysimetergefäßen anhand einer Zeichnung näher erläutert. Es zeigen:
- Fig. 1: eine schematische Draufsicht auf eine Lysimeteranordnung nach der Erfindung und
- Fig. 2: die Seitenansicht der Lysimeteranordnung nach Fig. 1.

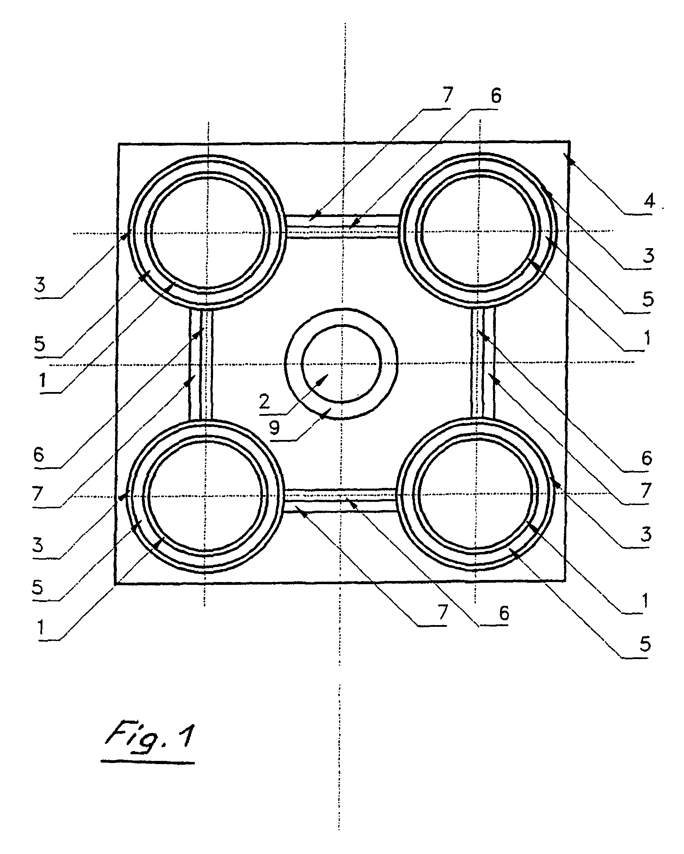

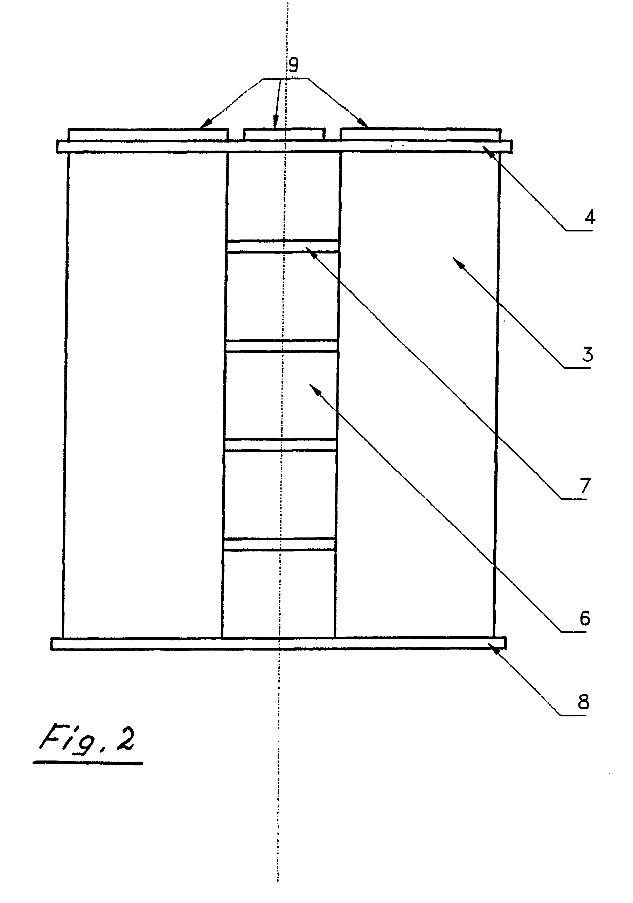

Die in der Fig. 1 dargestellte Lysimeterstation besteht aus vier wägbaren Lysimetergefäßen 1, die einen gemeinsamen Zugang 2 in der Mitte der kleeblattartigen Anordnung umschließen. Die Lysimetergefäße 1 sind jeweils in einem Zylinder 3 (Hüllrohr) eingebracht, die ihrerseits fest mit einer gemeinsamen Grundplatte 4 verbunden sind. Zwischen den Lysimetergefäßen 1 und den Zylindern 3 befindet sich ein Spalt 5, der so gewählt ist, daß die Lysimetergefäße 1 sich in den Zylindern 3 frei bewegen können und so eine Wägung gewährleisten. Zwischen den Zylindern 3 bzw. den Lysimetergefäßen 1, wenn es sich um eine nichtwägbare Lysimeterstation handelt, bei der die Zylinder 3 auch die Lysimetergefäße bilden, sind in mehreren Ebenen Stege 6, 7 zur Stabilisierung der Anordnung vorgesehen (Fig. 2).

Wie aus der Fig. 2 zu ersehen ist, sind der Zugang 2 und die Lysimetergefäße 1 und Zylinder 3 mit einer Deckplatte 8 gemeinsam abgedeckt. Die Deckplatte 8 weist einen nach oben gerichteten Rand 9 auf, so daß eine Begrünung der Abdeckung möglich ist und eine "Oasenbildung" mit den daraus resultierenden Nachteilen für das Mikroklima vermieden wird . Die Deck- und die Grundplatte 8,4 haben die gleichen Außenabmessungen. In der Deckplatte 8 ist ein nicht dargestellter Einstieg zum Zugang 2 vorgesehen. Die Deckplatte 8 ist ebenfalls mit den Lysimetergefäßen 1 und dem Zugang 2 fest verbunden.

Die Lysimetergefäße 1 weisen einen in geeigneter Weise ausgeführten Boden auf, der das Ablaufen von Sickerwasser gewährleistet. Es kann zum Beispiel ein Schrägboden eingearbeitet sein (nicht dargestellt), über den Sickerwasser zur Mitte des Gefäßes 1 geleitet und dort über z.B. ein Drainrohr abgelassen wird.
Eine solche Lysimeterstation ist aufgrund der Materialwahl auf jedem Standort einsetzbar und kann nach den Anwendungsfällen dimensioniert werden. Kleinere Lysimeterstationen sind wie Container über das Straßennetz transportierbar. Größere Lysimeterstationen können am Einsatzort zur vollen Größe zusammengeschweißt werden. Lysimeterstationen können auch modulartig aus einzelnen Einheiten zusammengefügt werden.

Es kann zweckmäßig sein, die Lysimeterstation auf eine Betonplatte zu setzen.

Die Erfindung ist nicht auf das hier beschriebene Ausführungsbeispiel beschränkt. Vielmehr ist es möglich, durch Kombination und Modifikation der beschriebenen Merkmale weitere Ausführungsvarianten zu realisieren, ohne den Rahmen der Erfindung zu verlassen.

### Bezugszeichenliste

- 1: Lysimetergefäß
- 2: Zugang
- 3: Zylinder
- 4: Grundplatte
- 5: Spalt
- 6: Steg
- 7: Steg
- 8: Deckplatte
- 9: Steg

## Patentansprüche

1. Lysimeterstation mit einem Zugang für die Funktionskontrolle sowie für die Aufnahme der Meß-, Steuerungs- und Wägetechnik,
**dadurch gekennzeichnet, daß**
der Zugang (2) mit mindestens einem Lysimetergefäß (1) einstückig als oben offener und zum Boden und den Seitenwänden hermetisch abgeschlossener Behälter ausgeführt ist.

2. Lysimeterstation nach Anspruch 1, **dadurch gekennzeichnet, daß** um den Zugang (2) vier Lysimetergefäße (1) kleeblattartig angeordnet sind.

3. Lysimeterstation nach den Ansprüchen 1 und 2, **dadurch gekennzeichnet, daß** die Lysimetergefäße (1) in Zylinder (3), die wenig größer als die Lysimetergefäße (1) ausgeführt sind und mit dem Zugang (2) einstückig verbunden sind, wägbar eingebracht sind.

4. Lysimeterstation nach den Ansprüchen 1 bis 3, **dadurch gekennzeichnet, daß** zwischen den Lysimetergefäßen (1) oder den Zylindern (3) und dem Zugang (2) Stege (6,7) zur Stabilisierung vorgesehen sind.

5. Lysimeterstation nach den Ansprüchen 1 bis 4, **dadurch gekennzeichnet, daß** für die Zylinder (3) oder Lysimetergefäße (1) und den Zugang (2) eine gemeinsame Abdeckung (8) mit einem nach oben gerichteten Steg (9) vorgesehen ist.

6. Lysimeterstation nach den Ansprüchen 1 bis 4, **dadurch gekennzeichnet, daß** die Anordnung der Lysimetergefäße (1) oder Zylinder (3) mit dem Zugang (2) in der Art eines Containers transportfähig ist.

7. Lysimeterstation nach den Ansprüchen 1 bis 6, **dadurch gekennzeichnet, daß** zur Herstellung ein inerter Kunststoff verwendet ist.

8. Lysimeterstation nach den Ansprüchen 1 bis 7, **dadurch gekennzeichnet, daß** die Lysimetergefäße (1) oder die Zylinder (3) und der Zugang (2) mit einer gemeinsamen Grundplatte (4) und der Deckplatte (8) fest verbunden sind.

## Claims

1. Lysimeter station with an access for the functional inspection as well as for the accommodation of the measuring, control and weighing technical devices,
wherein
the access (2) is executed with at least one lysimeter vessel (1), in one piece as a vessel which is open on top and which is hermetically closed to the bottom and to the side walls.

2. Lysimeter station according to Claim 1, **wherein** four lysimeter vessels (1) are located in a clover-type arrangement around the access (2).

3. Lysimeter station according to Claims 1 and 2, **wherein** the lysimeter vessels (1) are weighably located in cylinders (3), executed a little larger than the lysimeter vessels (1), and joined with the access (2) in a one-piece union.

4. Lysimeter station according to Claims 1 to 3, **wherein** ligaments (6, 7) are provided for stabilisation purposes between the lysimeter vessels (1) or the cylinders (3) and the access (2).

5. Lysimeter station according to Claims 1 to 4, **wherein** a common covering (8) with an upward orientated ligament (9) is provided for the cylinders (3) or lysimeter vessels (1) and the access (2).

6. Lysimeter station according to Claims 1 to 4, **wherein** the arrangement of the lysimeter vessels (1) or the cylinders (3) with the access (2) is transportable in the mode of a container.

7. Lysimeter station according to Claims 1 to 6, **wherein** an inert synthetic material is used for the manufacture.

8. Lysimeter station according to Claims 1 to 7, **wherein** the lysimeter vessels (1) or the cylinders (3) and the access (2) are solidly connected with a common base plate (4) and the cover plate (8).

## Revendications

1. Station de lysimètre avec un accès pour le contrôle de fonctionnement ainsi que pour recevoir la technique de mesure de commande et de pesage, **caractérisé en ce que** l'accès (2) est réalisé avec au moins un réservoir de lysimètre (1) en une pièce en tant que réservoir ouvert en haut et hermétiquement fermé vers le fond et vers les côtés latéraux.

2. Station de lysimètre selon la revendication 1, **caractérisé en ce que** quatre réservoirs de lysimètre (1) sont disposés en forme de feuille de trèfle autour de l'accès (2).

3. Station de lysimètre selon les revendications 1 et 2, **caractérisé en ce que** les réservoirs de lysimètre (1) sont introduits de façon pesable dans des cylindres (3) qui sont réalisés un peu plus grand que les réservoirs de lysimètre (1) et qui sont reliés de façon solidaire avec l'accès (2).

4. Station de lysimètre selon les revendications 1 à 3, **caractérisé en ce que** des traverses (6,7) sont prévues pour la stabilisation entre les réservoirs de lysimètre (1) ou les cylindres (3) et l'accès (2).

5. Station de lysimètre selon les revendications 1 à 4, **caractérisé en ce qu'**une couverture commune (8) avec une traverse 9 orientée vers le haut, est prévue pour les cylindres (3) ou les réservoirs de lysimètre (1) et l'accès (2).

6. Station de lysimètre selon les revendications 1 à 4, **caractérisé en ce que** la disposition des réservoirs de lysimètre (1) ou les cylindres (3) avec l'accès (2) peuvent être transportés comme un conteneur.

7. Station de lysimètre selon les revendications 1 à 6, **caractérisé en ce qu'**une matière plastique inerte est utilisée pour la fabrication.

8. Station de lysimètre selon les revendications 1 à 7, **caractérisé en ce que** les réservoirs de lysimètre (1) ou cylindres (3) et l'accès (2) sont liés de manière fixe au moyen d'une plaque de base commune (4) et au moyen d'une plaque de couvercle (8).
